# EUROPÄISCHE PATENTSCHRIFT

(11) **EP 0 651 989 B1**
(45) Veröffentlichungstag und Bekanntmachung des Hinweises auf die Patenterteilung: **24.02.1999**
(21) Anmeldenummer: 94250267.5
(22) Anmeldetag: 01.11.1994
(51) Int. Cl.: A61K 6/083

(54) **Mehrkomponentendentalzement auf Calciumhydroxid-Basis**
Calciumhydroxide based multicomponent dental cement
Ciment dentaire à plusieurs constituants à base d'hydroxyde de calcium

(30) Priorität: 10.11.1993 DE 4339009
(43) Veröffentlichungstag der Anmeldung: 10.05.1995
(73) Patentinhaber: IVOCLAR AG, FL-9494 Schaan (LI)
(72) Erfinder: Rheinberger, Volker, Dr., FL-9490 Vaduz (LI); Moszner, Norbert, Prof. Dr., FL-9492 Eschen (LI); Salz, Ulrich, Dr., D-88138 Weissensberg (DE)
(74) Vertreter: UEXKÜLL & STOLBERG

(56) Entgegenhaltungen:
- EP-A- 0 189 903
- WO-A-93/16676
- DE-A- 3 801 207
- DE-A- 4 141 174

## Beschreibung

Die vorliegende Erfindung betrifft ein Mehrkomponentendentalzementsystem auf Calciumhydroxid-Basis, das als eine erste Komponente eine ethylenisch ungesättigte, polymerisierbare Verbindung, als eine zweite Komponente Calciumhydroxid oder Calciumoxid und außerdem einen Polymerisationsinitiator umfaßt. Der Zement härtet durch Chelatisierung der Calciumionen und radikalische Polymerisation der ethylenisch ungesättigten Monomere aus.

In der Zahnmedizin wird eine Reihe von sogenannten Dentalzementen beispielsweise zum Befestigen von Kronen, Inlays oder kieferorthopädischen Vorrichtungen, als Wurzelkanalfüllungsmaterial, als Unterfüllungsmaterial bei der Einbringung von dentalem Restaurationsmaterial oder auch als Füllungsmaterial selbst verwendet. Dentalzemente bestehen beispielsweise aus einer Mischung von feinteiligen Metalloxiden oder Metallhydroxiden, die mit einer Anrührflüssigkeit, die im wesentlichen Phosphorsäuren, Polycarbonsäuren oder auch Salicylsäurederivate enthält, zur Reaktion gebracht wird.

Ein solches Metallhydroxid ist beispielsweise Calciumhydroxid. Calciumhydroxid wird u.a. wegen seiner bakteriziden Wirkung und seiner stimulierenden Wirkung auf die Sekundärdentin-Bildung in der Zahnmedizin schon seit mehr als 50 Jahren eingesetzt (Hermann B.W.: Zahnärztliche Rundschau 1930, 39, S. 888). Hauptsächlich wurden bzw. wird es in Form einer wäßrigen Aufschlämmung als Unterfüllungsmaterial zur Pulpenüberkappung oder als temporäre Wurzeleinlage verwendet. Der Nachteil von wäßrigen Calciumhydroxidaufschlämmungen besteht darin, daß dieser Materialtyp keine Abbindereaktion zeigt und somit auch mechanisch kaum belastbar ist. Deshalb sind Calcium-hydroxidaufschlämmungen nur in dünnen Schichten bzw. temporär einsetzbar.

Eine Abbindereaktion und somit auch eine gewisse mechanische Belastbarkeit zeigen dagegen Calciumhydroxid-Zemente, die, wie in der US-A-3 047 408 beschrieben, Salicylate enthalten. Es wird dabei ein Überschuß an Calciumhydroxid mit einem Salicylsäureester eines mehrwertigen Alkohols zur Reaktion gebracht, d.h. bei der Abbindereaktion wird das Ca²⁺-Ion vom Salicylat komplexiert, wobei sich eine polymere Netzwerkstruktur ausbildet. Neben der immer noch relativ niedrigen mechanischen Belastbarkeit zeigen derartige Zemente jedoch weitere Nachteile. Zum einen läuft die Abbindereaktion sehr langsam ab und ist sehr feuchtigkeitsempfindlich, d.h. dieser Materialtyp zeigt keine sogenannte Kommandoaushärtung, was bei der Verarbeitung, z.B. dem Anbringen einer Unterfüllung, recht wichtig ist, damit sofort nach dem Legen der Unterfüllung mit dem Aufbau der eigentlichen Füllung begonnen werden kann. Zum anderen fällt dieser Materialtyp durch einen sehr intensiven Eigengeruch auf.

Zur Realisierung der Kommandohärtung und zur Verstärkung der mechanischen Belastbarkeit wurden in den letzten Jahren lichthärtende Zemente auf den Markt gebracht. Diese Zemente enthalten in allen Fällen Verbindungen mit einer Methacrylatgruppe, welche mit einem im sichtbaren Bereich absorbierenden Lichtkatalysator durch Bestrahlung polymerisiert werden.

In der EP-A-0 189 903 und der DE-A-38 01 207 sind beispielsweise derartige Systeme beschrieben. Gemäß der EP-A-0 189 903 wird ein Unterfüllungsmaterial mit einem Gehalt an Calciumhydroxid oder einem Calciumhydroxid bildenden Mittel wie Calciumoxid beschrieben, welches photopolymerisierbar ist. Dieses Unterfüllungsmaterial enthält ethylenisch ungesättigte Verbindungen, wozu insbesondere Vinylurethan- oder Urethan(meth)acrylatverbindungen gehören, einen Photokatalysator, z.B. Campherchinon, in Kombination mit einem Amin. Das Calciumhydroxid wird dabei nicht über eine Chelatisierung sondern rein physikalisch durch Einschluß in das Polymernetzwerk gebunden. Das entsprechende Produkt ist unter dem Namen "Prisma Dycal VLC" auf den Markt gekommen. In der Literatur wird berichtet (Staehle, H.J., Calciumhydroxid in der Zahnheilkunde, Hanser, München 1990), daß Prisma Dycal VLC bedingt durch seine geringe Löslichkeit nur noch einen minimalen antimikrobiellen Effekt zeigt. Ferner werden nur geringe Aushärtungstiefen erreicht. Gemäß der DE-A-38 01 207 werden Verbindungen eingesetzt, in denen eine Methacrylatfunktion mit einer Salicylatfunktion in einem Molekül vereint ist. Es wird allgemein angenommen, daß das Aushärten der zementartigen Auskleidung über eine Chelatisierung der Calciumionen unter Ausbildung einer ionischen Gitterstruktur mit dem Salicylat erfolgt. Neben dem auch bei diesem System vorhandenen Nachteil des starken Eigengeruchs besteht die Gefahr, daß durch die phenolische Salicylatgruppe die radikalische Polymerisation inhibiert wird. In der Literatur findet man häufig Berichte darüber (siehe z.B. Millstein, P.L., Nathanson, D., J. Prosthet. Dent. 1983. 50, 211), daß die Polymerisation von Füllungskomposits auf Methacrylatbasis durch phenolische Verbindungen (speziell Eugenol) gestört wird.

Ferner sind aus der EP-A-0 219 058 polymerisierbare Zementmischungen bekannt, die a) polymerisierbare, ungesättigte Monomere und/oder Oligomere und/oder Polymere mit Säuregruppen und/oder reaktiven Säurederivatgruppen, b) feinteilige, reaktive Füllstoffe, die mit diesen Säuren oder Säurederivaten reagieren können, wie Calciumhydroxid und c) Härtungsmittel enthalten, wobei die an den polymerisierbaren Verbindungen vorhandenen Säuregruppen oder Säurederivatgruppen mit den feinteiligen reaktiven Füllstoffen ionisch zu einer Zementreaktion zu führen vermögen. Diese Zementzusammensetzungen weisen jedoch den Nachteil auf, daß insbesondere die beschriebenen präpolymeren Polysäuren zusätzlich synthetisiert und aufwendig gereinigt werden müssen. Darüber hinaus läßt sich mit zunehmendem Polymerisationsgrad der Polysäuren weniger Füllstoff einarbeiten.

Brauer, G.M., White, E.E., Moshonas, M.G., J. Dent. Res. 1958, 37, 547 und Nielsen, T.L., Acta Odont, Scand. 1963, 21, 159, beschreiben die Verwendung von Acetylaceton oder Acetessigsäureethylester in Kombination mit ZnO als Dentalzement, fanden aber, daß solche Kombinationen unbrauchbar waren.

In der DE-A-41 41 174 sind härtbare Massen für die dentale Wiederherstellung beschrieben, die dadurch gekennzeichnet sind, daß sie ein Vinylmonomer, das nicht weniger als 30 Gew.% eines Säuregruppen enthaltenden Vinylmonomeren der Formel (1) enthält, einen Ionen-mineralisierbaren Füllstoff und einen Polymerisationsinitiator enthalten.

Aus der WO-A-93 16676 sind Ionomer-Polymere und Ionomer-Polymerzemente bekannt, die β-Dicarbonylgruppen enthalten. Die Polymere werden in Kombination mit reaktiven Glaspulvern und in Gegenwart von Wasser umgesetzt. Neben einer Komplexierung der β-Dicarbonylgruppen erfolgt die Härtung der Ionomere auf Basis von Acrylsäure und β-Ketoestern aufgrund der gut bekannten Glasionomerbildung und damit aufgrund einer ionischen Vernetzung, so daß wiederum eine klassische Säure/BaseReaktion vorliegt.

Der Erfindung liegt die Aufgabe zugrunde, einen Mehrkomponentendentalzement auf Calciumoxid- oder Calciumhydroxid-Basis zur Verfügung zu stellen, der die oben beschriebenen Nachteile nicht aufweist und verbesserte physikalische Eigenschaften aufweist.

Diese Aufgabe wird durch einen Mehrkomponentendentalzement gelöst, der als eine erste Komponente eine ethylenisch ungesättigte, polymerisierbare Verbindung, als eine zweite Komponente Calciumhydroxid oder Calciumoxid und außerdem einen Polymerisationsinitiator umfaßt, dessen Bestandteile in einer oder mehreren Zementkomponenten enthalten sein können, und dadurch gekennzeichnet ist, daß die ethylenisch ungesättigte, polymerisierbare Verbindung sowohl mindestens eine β-Dicarbonylgruppe als auch mindestens eine Acryl-, Alkacryl- oder vinylaromatische Gruppe aufweist, wobei der Zement durch Chelatisierung der Calciumionen durch die β-Dicarbonylverbindung und radikalische Polymerisation der ethylenisch ungesättigten Monomere aushärtet.

Der Zement kann insbesondere mehr als eine dieser ethylenisch ungesättigten, polymerisierbaren Verbindungen enthalten.

Die ethylenisch ungesättigte, polymerisierbare Verbindung kann auch mehrere β-Dicarbonylgruppen und/oder mehrere Acryl-, Alkacryl- oder vinylaromatische Gruppen aufweisen, z.B. jeweils 2 bis 4.

Bei der Alkacrylgruppe handelt es sich vorzugsweise um eine mit einer C₁- bis C₁₀-Alkylguppe, vorzugsweise C₁- bis C₅-Alkylguppe substituierte Acrylgruppe und insbesondere um eine Methacrylgruppe und die vinylaromatische Gruppe ist vorzugsweise eine Styryl bzw. 4-Vinylbenzylgruppe.

Das Verhältnis der β-Dicarbonylgruppen zu den ethylenisch ungesättigten, polymerisierbaren Gruppen liegt vorzugsweise im Bereich von 5 : 1 bis 1 : 5, insbesondere 3 : 1 bis 1 : 3 und ist am meisten bevorzugt 1 : 1.

Gemäß einer bevorzugten Ausführungsform ist die ethylenisch ungesättigte, polymersierbare Verbindung eine Verbindung der Formel 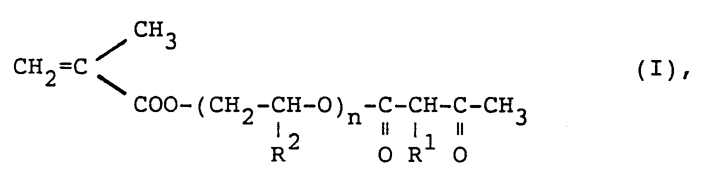 in der
- n =: 1 bis 20, insbesondere 1 bis 10 ist,
- R¹ =: Wasserstoff oder eine C₁- bis C₁₀-Alkylgruppe, insbesondere Wasserstoff oder eine C₁- bis C₅-Alkylgruppe wie eine Methylgruppe ist, und
- R² =: Wasserstoff oder eine C₁- bis C₁₀-Alkylgruppe, insbesondere Wasserstoff oder eine C₁- bis C₅-Alkylgruppe wie eine Methyl- oder Ethylgruppe,
oder der Formel 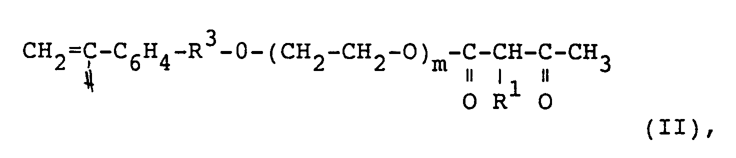 ist, in der
- m =: 0 bis 20, insbesondere 0 bis 10 ist,
- R¹ =: Wasserstoff oder eine C₁- bis C₁₀-Alkylgruppe, insbesondere Wasserstoff oder eine C₁- bis C₅-Alkylgruppe wie eine Methylgruppe ist, und
- R³ =: eine C₁- bis C₁₀-Alkylengruppe, insbesondere eine C₁- bis C₅-Alkylengruppe wie eine Methylengruppe ist.

Zu diesen bevorzugten Verbindungen gehören insbesondere 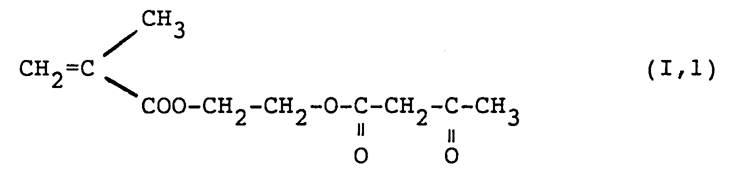 (Acetoacetoxyethylmethacrylat, AAEMA) 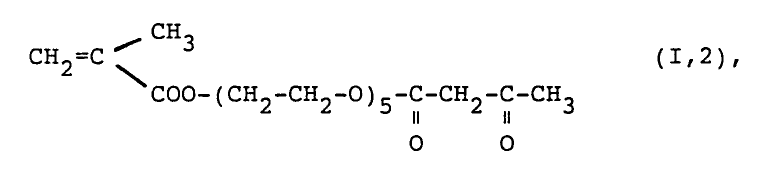 (Acetoacet-PEG-200-methacrylat, PEG-200-AAMA)
oder 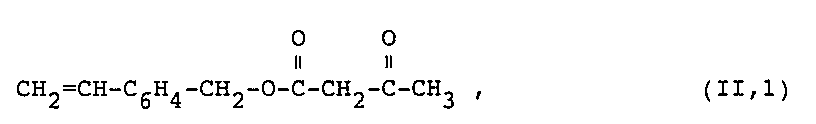 (Acetessigsäure-4-vinylbenzylester, AASt).

Die erfindungsgemäßen β-Dicarbonylverbindungen der Formeln I und II lassen sich beispielsweise nach dem in der DE-A-21 56 446 beschriebenen Verfahren, das gegebenenfalls an die speziell eingesetzten Verbindungen anzupassen ist, herstellen. So erhält man die Verbindung (I, 1) in nahezu quantitativer Ausbeute, wenn man 1 Mol der entsprechenden Hydroxyverbindung, 1 g Triethylamin und 1 g 2,6-Di-tert.-Butyl-p-kresol in 500 ml absolutem Ethylacetat löst, zu dieser Lösung innerhalb 1 Stunde unter Rühren 88 g (1,05 Mol) Diketen tropft, das Reaktionsgemisch anschließend ca. 2 Stunden lang unter Rückfluß erhitzt, nach dem Abkühlen zuerst mit verdünnter HCl und anschließend mit Wasser wäscht und nach dem Trocknen mit Natriumsulfat das Lösungsmittel abdestilliert (siehe z.B. auch DE-A-31 49 797). Die Verbindung (I, 1) ist darüber hinaus kommerziell von der Firma Lonza AG in 96 %-iger Reinheit erhältlich.

Analog können die Verbindungen (I, 2) und (II, 1) durch Umsetzung von Diketen mit Polyethylenglykol (PEG)-200-Monomethacrylat bzw. 4-Vinylbenzylalkohol in Gegenwart von 1,4-Diazabicyclo(2.2.2)octan (DABCO) hergestellt werden. Ein entsprechendes Verfahren ist auch in der GB-A-1 144 486 beschrieben.

Der erfindungsgemäße Mehrkomponentendentalzement enthält die ethylenisch ungesättigte, polymerisierbare Verbindung, vorzugsweise in einer Menge von 7 bis 70, insbesondere 7 bis 60 und bevorzugt 7 bis 55 Gew-.%.

Überdies können üblicherweise in derartigen Zementen verwendete Zusatzstoffe vorhanden sein. Hierzu gehören Monomere, Initiatoren und Beschleuniger sowie Stabilisatoren und Füllstoffe.

Als Beispiele für Monomere kommen Methylmethacrylat, Triethylenglykoldimethacrylat, Hexandioldimethacrylat, Dodecandioldimethacrylat, Bisphenol-A-dimethacrylat, Bisphenol-A-glycidylmethacrylat, Trimethylolpropantrimethacrylat, Hydroxethylmethacrylat und Urethandimethacrylate in Frage, z.B. die im folgenden als Urethandimethacrylat bezeichneten Umsetzungsprodukte von 1 Mol Hexamethylendiisocyanat bzw. Trimethylhexamethylendiisocyanat mit 2 Mol 2-Hydroxyethylmethacrylat oder von 1 Mol Tri(6-isocyanatohexyl)biuret mit 3 Mol 2-Hydroxyethylmethacrylat.

Als Initiatoren für die Heißpolymerisation können die bekannten Peroxide wie Dibenzoylperoxid, Dilaurylperoxid, tert.-Butylperoctoat oder tert.-Butylperbenzoat eingesetzt werden, aber auch Azobisisobutyroethylester, Benzpinakol oder 2,2'-Dimethylbenzpinakol sind geeignet.

Als Initiatoren für die Photopolymerisation können z.B. Benzophenon und Benzoin sowie deren Derivate verwendet werden. Darüber hinaus können auch α-Diketone wie 9,10-Phenanthrenchinon, Diacetyl oder 4,4'-Dichlorbenzil eingesetzt werden. Campherchinon (CC) wird bevorzugt verwendet, wobei die α-Diketone in Kombination mit Aminen wie Cyanoethylmethylanilin, Dimethylaminoethylmethacrylat, Triethanolamin oder N,N-Dimethyl-sym.-xylidin als Reduktionsmittel besonders bevorzugt sind.

Als Initiatoren für die Kaltpolymerisation werden Radikale liefernde Systeme, z.B. Benzoyl- (BP) bzw. Laurylperoxid zusammen mit Aminen wie N,N-Dimethyl-sym.-xylidin, N,N-Dimethyl-p-toluidin oder 3,5-Di-tert.-butyl-N,N'-diethylanilin (DABA) verwendet.

Als normale Füllstoffe eignen sich beispielsweise amorphe Kieselsäuren, insbesondere die pyrogene und gefällte Kieselsäure mit einer BET-Oberfläche von 30 bis 300 m²/g (Aerosil 200, Ox 50) oder Zinkoxid (ZnO), während als röntgenopake Füllstoffe röntgenopake Gläser, Bariumsulfat oder Ytterbiumfluorid bevorzugt Verwendung finden und die anorganischen Bestandteile in der üblichen Weise mit z.B. 3-Methacryloyloxypropyltrimethoxysilan silanisiert sein können (Ox 50 sil.).

Dem Dentalmaterial können ferner feinteilige Splitter- oder Perlpolymerisate einverleibt werden. Diese Homo- bzw. Copolymeren von den üblichen mono- oder polyfunktionellen Methacrylaten können ihrerseits mit den beschriebenen anorganischen Füllstoffen, auch den röntgenopaken, gefüllt sein.

Zur Verbesserung der Lagerstabilität setzt man den Materialien Stabilisatoren zu wie beispielsweise Hydrochinonmonomethylether (MEHQ) oder 2,6-Di-tert.-butyl-4-methylphenol (BHT).

Als Polymerisationsinitiatoren sind übliche Polymerisationsinitiatoren geeignet, wozu auch Photoinitiatoren gehören. Beispiele geeigneter Polymerisationsinitiatoren sind Benzoylperoxid und Campherchinon.

Bei der Umsetzung des Calciumhydroxids oder des Calciumoxids mit der erfindungsgemäßen ethylenisch ungesättigten, polymerisierbaren β-Dicarbonylverbindung werden die entsprechenden Calciumkomplexe gebildet.

Um diese Calciumkomplexe charakterisieren zu können, wurden sie gemäß folgendem Verfahren hergestellt und isoliert. Es wurden 20 mMol Ca-Verbindung in 70 ml Wasser/Ethanol (1 : 1) suspendiert (Calciumhydroxid, Calciumcarbonat oder Calciumphosphat) bzw. gelöst (Calciumacetat oder Calciumchlorid). Dazu wurden unter Rühren bei Raumtemperatur 10 ml einer ethanolischen Lösung von 40 mMol AAEMA getropft.

Im Fall von Calciumchlorid, -carbonat, -phosphat und -acetat wurden nochmals 10 ml einer 2,0 molaren Ammoniaklösung zugegeben. Nach weiterem 2-stündigem Rühren wurde der gebildete Niederschlag abfiltriert, gründlich mit Wasser und Ethanol gewaschen und im Feinvakuum getrocknet. Die Ausbeute betrug bei Verwendung von Calciumhydroxid 73,3 %, von Calciumchlorid 31,4 % bzw. von Calciumacetat 29,4 %. Analog dazu erfolgte als Vergleichsbeispiel die Umsetzung von Calciumhydroxid mit Acetessigester (EAA) mit einer Ausbeute von 68,8 %. Ferner wurde analog die Umsetzung von Calciumhydroxid mit PEG-200-AAMA durchgeführt, wobei die gebildeten Komplexe sowohl in Wasser als auch in organischem Lösungsmittel sehr gut löslich und damit schwer isolierbar waren.

Darüber hinaus war der Ca-AAEMA-Komplex analog der Literaturvorschrift von West, R., Riley, R., J. Inorg. Nucl. Chem. 1958, 5, 295, in 94,1 %-iger Ausbeute zugänglich, wobei 0,10 Mol metallisches Calcium in 350 ml absolutem Ethanol durch Erwärmen unter Rückfluß gelöst wurden. Nach Abkühlen auf Raumtemperatur wurden 50 ml einer Lösung von 0,20 Mol AAEMA in Ethanol zugetropft und 2 Stunden gerührt. Der entstandene Niederschlag wurde abfiltriert bzw. gewaschen und schließlich im Feinvakuum getrocknet. Der Ca-AAEMA-Komplex ist in DMF bzw. Dimethylsulfoxid (DMSO) löslich.

Die gewonnenen Ca-Komplexe wurden durch Schmelzpunkt, Elementaranalyse, Glührückstand, ¹H-NMR- und IR-Spektroskopie charakterisiert.

### Ca(AAEMA)₂:

Fp.: 189-191°C
CaC₂₀H₂₆O₁₀ (466,5): gef.; C: 50,26 (ber.: 51,50), H: 5,66 (5,62);
Glührückstand: 12,74 (ber.: 12,02 CaO);
¹H-NMR (DMSO-d₆): 1,72 (3H,s,CH₃-C(O⁻)=), 1,90 (3H,s,CH₃-C=), 4,03-4,33 (4H,m,-CH₂-CH₂),4,53 (1H,s,=CH-), 5,70 (1H,s) und 6,07 ppm (1H,s,CH₂=);
IR (KBr): 1719 (C=O), 1646 (C=O), 1530 cm⁻¹ (C=C).

### Ca(EAA)₂: (Vergleichsbeispiel)

Glührückstand: 18,80 (19,13 CaO);
¹H-NMR (DMSO-d₆): 1,22 (3H,t,-CH₂-CH₃), 1,71 (3H,s,CH₃-C(O⁻)=), 3,92 (2H,q,CH₂-CH₃-), 4,49 ppm (1H,s,=CH-);
IR (KBr): 1642 (C=O), 1518 cm⁻¹ (C=C).

In dem folgenden Beispiel werden erfindungsgemäße Dentalzement-Formulierungen beschrieben, die AAEMA oder PEG-200-AAMA als erfindungsgemäße β-Dicarbonylverbindung enthalten, wobei sich die einzelnen Formulierungen durch einen unterschiedlichen Gehalt an diesen Verbindungen voneinander unterscheiden.

Der Calciumhydroxid-Dentalzement wurde gemäß folgender Rezeptur hergestellt.

### Basenpaste:

| | |
|---|---|
| Urethandioldimethacrylat | 29,6 Masse-% |
| Butandioldimethacrylat | 7,3 Masse-% |
| DABA | 0,3 Masse-% |
| MEHQ | 0,1 Masse-% |
| Ca(OH)₂ | 43,0 Masse-% |
| ZnO | 5,4 Masse-% |
| Ox 50 sil. | 14,3 Masse-% |

### Aktivatorpaste:

| | |
|---|---|
| AAEMA bzw. AAEMA/Urethandimethacrylatmischung | 53,9 Masse-%* |
| 53,90 % AAEMA bei 100 % Anteil; | |
| 26,95 % AAEMA bei 50 % Anteil; | |
| 8,10 % AAEMA bei 15 % Anteil; | |
| BaSO₄ | 39,9 Masse-% |
| Aerosil 200 | 3,9 Masse-% |
| Benzoylperoxid 50-FT | 2,0 Masse-% |
| Campherchinon | 0,3 Masse-% |

| | |
|---|---|
| * Das AAEMA wurde rein (100 %) bzw. in Abmischung mit Urethandimethacrylat (50 %, 15 %) eingesetzt. In einem weiteren Fall wurde PEG-200-AAMA in 50 %-iger Abmischung mit Urethandimethacrylat einesetzt. | |

Die entsprechenden Massen wurden in üblicher Weise durch Mischen in einem Kneter hergestellt. Die dabei erhaltenen Dentalzemente wurden anschließend charakterisiert. Dazu wurden Basen- und Aktivatorpaste in einem Volumenverhältnis von 1 : 1 angemischt. Die erhaltenen Werte sind in Tabelle 1 aufgeführt.

**Tabelle**

| **Materialeigenschaften von Dentalzement-Varianten mit AAEMA oder PEG-200-AAMA als Komplexierungsmittel oder BASIC L (Vergleich) nach Wasser- (WL) bzw. Milchsäurelagerung (ML)** | | | | | | | | |
|---|---|---|---|---|---|---|---|---|
| | | % AAEMA in der Monomermischung der Aktivatorpaste | | | | | Basic L | Prisma VLC Dycal® |
| | | 100 | 50 | 50^{b} | 15 | 50^{c} | | |
| Biegefestigkeit (MPa): | 24 h WL | - | 40,40 | - | 74,80 | 34,70 | 13 | 29 |
| E-Modul (MPa): | 24 h WL | - | 2017 | - | 4411 | 1427 | - | 910 |
| Druckfestigkeit (MPa): | ohne WL^{d} | - | 152,10 | 173,30 | - | - | - | - |
| | 24 h WL | 59,30 | 148,70 | 142,40 | 197,10 | 115,60 | 75 | 180 |
| | 7 d WL | 24,00 | 131,10 | 55,60 | 199,40 | 49,90 | 76 | 190 |
| | 24 h ML | 51,00 | 173,30 | - | - | - | - | - |
| | 7 d ML | 22,80 | 190,50 | - | - | - | - | - |
| Wasseraufnahme (%) | | 29,7 | 12,8 | - | 1,8 | 13,1 | - | - |
| Wasserlöslichkeit (%) | | 37,2 | 12,7 | - | 0,4 | 9,3 | 0,35 | - |
| Säurelöslichkeit (mm) | | 0,024 | 0,014 | - | unlösl. | unlös. | 0,03 | - |

| | | | | | | | | |
|---|---|---|---|---|---|---|---|---|
| ^{b} Ohne Belichtung; | | | | | | | | |
| ^{c} statt AAEMA Einsatz von PEG-200-AAMA; | | | | | | | | |
| ^{d} BASIC L (Vergleich) Druckfestigkeit: 75 MPa (24 h WL), 76 MPa (7 d WL). | | | | | | | | |

### Zusammensetzung von Basic L:

| Base: | Katalysator: |
|---|---|
| 29,4 Gew.% Urethandimethacrylat | 24,0 Gew.% 1,3-Butandiolsalicylat |
| 7,3 Gew.% Butandioldimethacrylat | 24,0 Gew.% Trimethylhexandioldisalicylat |
| 43,3 Gew.% Ca(OH)₂ | |
| 14,2 Gew.% Ox 50 | 6,0 Gew.% Methylsalicylat |
| 5,4 Gew.% ZnO | 40,0 Gew.% Barium sulfat |
| 0,3 Gew.% DABA | 3,9 Gew.% Aerosil 200 |
| 0,1 Gew.% MEHQ | 2,0 Gew.% BP |
| | 0,1 Gew.% CC |
| (Das Mischungsverhältnis Base/Katalysator = 1:1) | |

### Zusammensetzung von Prisma VCL Dycal®:

| |
|---|
| 61,6 Gew.% Monomer |
| 18,9 Gew.% Bariumsulfat |
| 18,9 Gew.% Ca(OH)₂ |

Die Ergebnisse belegen die verbesserten physikalischen Eigenschaften der erfindungsgemäß hergestellten Calciumhydroxidzemente, insbeson-dere die verbesserte Biegefestigkeit und den verbesserten E-Modul sowie insgesamt gesehen, daß die mechanische Festigkeit dieser Zemente brauchbar ist.

Die Verarbeitungsbreite der erfindungsgemäßen Dentalzemente liegt zwischen 40 und 80 Sekunden und die Durchhärtungstiefe bei 6 mm (40 Sek. Bestrahlungszeit; Heliomat (Vivadent); 80 % AAEMA).

Im Vergleich hierzu:
- Basic L: 2,0 mm (40 Sek. Bestrahlungszeit)
- Prisma VCL Dycal®: 1,4 mm (40 Sek. Bestrahlungszeit)

Eine Haftung am Dentin wird nicht beobachtet. Dies ist dadurch erklärbar, daß die β-Dicarbonylverbindungen mit dem vorhandenen (Überschuß) Ca(OH)₂ ausreagieren, d.h. es findet eine vollständige Komplexierung statt.

## Patentansprüche

1. Mehrkomponentendentalzementsystem, das als eine erste Komponente eine ethylenisch ungesättigte, polymerisierbare Verbindung, als eine zweite Komponente Calciumhydroxid oder Calciumoxid und außerdem einen Polymerisationsinitiator umfaßt, dessen Bestandteile in einer oder mehreren Zementkomponenten enthalten sein können, dadurch gekennzeichnet, daß die ethylenisch ungesättigte, polymerisierbare Verbindung sowohl mindestens eine β-Dicarbonylgruppe als auch mindestens eine Acryl-, Alkacryl- oder vinylaromatische Gruppe aufweist, wobei der Zement durch Chelatisierung der Calciumionen durch die β-Dicarbonylverbindung und radikalische Polymerisation der ethylenisch ungesättigten Monomere aushärtet.

2. Mehrkomponentendentalzementsystem nach Anspruch 1, dadurch gekennzeichnet, daß die Alkacrylgruppe eine Methacrylgruppe ist und die vinylaromatische Gruppe eine Styrylgruppe ist.

3. Mehrkomponentendentalzementsystem nach Anspruch 1 oder 2, dadurch gekennzeichnet, daß das Verhältnis der β-Dicarbonylgruppen zu den ethylenisch ungesättigten, polymerisierbaren Gruppen im Bereich von 5:1 bis 1:5, insbesondere 3:1 bis 1:3 liegt, wobei 1:1 bevorzugt ist.

4. Mehrkomponentendentalzementsystem nach einem der Ansprüche 1 bis 3, dadurch gekennzeichnet, daß die ethylenisch ungesättigte, polymerisierbare Verbindung eine Verbindung der Formel 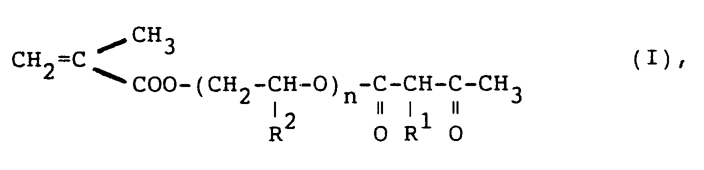 in der
n = 1 bis 20, insbesondere 1 bis 10 ist,
R¹ = Wasserstoff oder eine C₁- bis C₁₀-Alkylgruppe, insbesondere Wasserstoff oder eine C₁- bis C₅-Alkylgruppe wie eine Methylgruppe ist, und
R² = Wasserstoff oder eine C₁- bis C₁₀-Alkylgruppe, insbesondere Wasserstoff oder eine C₁- bis C₅-Alkylgruppe wie eine Methyl- oder Ethylgruppe ist,
oder der Formel 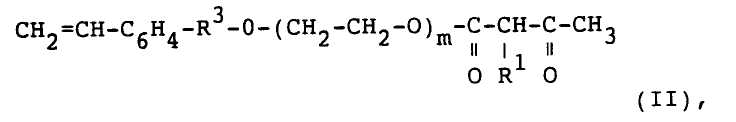 ist, in der
m = 0 bis 20, insbesondere 0 bis 10 ist,
R¹ = Wasserstoff oder eine C₁- bis C₁₀-Alkylgruppe, insbesondere Wasserstoff oder eine C₁- bis C₅-Alkylgruppe wie eine Methylgruppe ist, und
R³ = eine C₁- bis C₁₀-Alkylengruppe, insbesondere eine eine C₁- bis C₅-Alkylengruppe wie Methylengruppe ist.

5. Mehrkomponentendentalzementsystem nach Anspruch 4, dadurch gekennzeichnet, daß die ethylenisch ungesättigte, polymerisierbare Verbindung 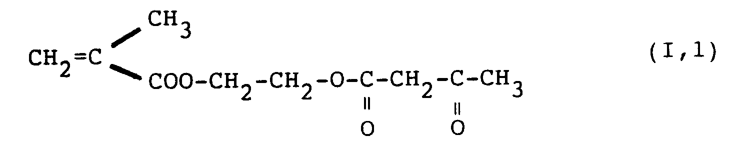 (Acetoacetoxyethylmethacrylat, AAEMA) 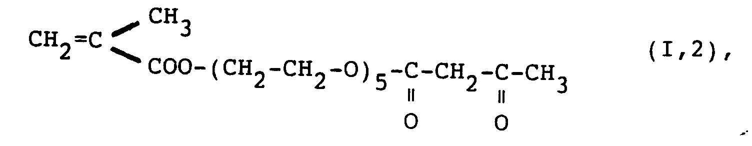 (Acetoacet-PEG-200-methacrylat, PEG-200-AAMA)
oder 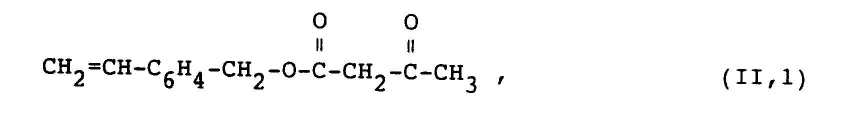 (Acetessigsäure-4-vinylbenzylester, AASt)
ist.

6. Mehrkomponentendentalzementsystem nach einem der Ansprüche 1 bis 5, dadurch gekennzeichnet, daß er zusätzlich übliche Zusatzstoffe, insbesondere Füllstoff umfaßt.

7. Mehrkomponentendentalzementsystem nach einem der Ansprüche 1 bis 6, dadurch gekennzeichnet, daß er die ethylenisch ungesättigte, polymerisierbare Verbindung in einer Menge von 7 bis 70, insbesondere 7 bis 60 und bevorzugt 7 bis 55 Gew.% enthält.

8. Mehrkomponentendentalzementsystem nach einem der Ansprüche 1 bis 7, dadurch gekennzeichnet, daß die ethylenisch ungesättigte, polymerisierbare Verbindung mehrere β-Dicarbonylgruppen und/oder mehrere Acryl-, Alkacryl- oder vinylaromatische Gruppen aufweist.

9. Verfahren zur Herstellung eines Mehrkomponentendentalzements gemäß einem der Ansprüche 1 bis 8, dadurch gekennzeichnet, daß zunächst a) eine Basispaste, die das Calciumhydroxid oder Calciumoxid enthält, und b) eine Aktivatorpaste, die die ethylenisch ungesättigte, polymerisierbare Verbindung enthält, separat hergestellt werden und diese beiden Pasten anschließend zusammengemischt werden.

10. Verwendung einer ethylenisch ungesättigten, polymerisierbaren Verbindung, die sowohl mindestens eine β-Dicarbonylgruppe als auch mindestens eine Acryl-, Alkacryl- oder vinylaromatische Gruppe aufweist, zur Herstellung eines Calciumhydroxid-Dentalzements.

## Claims

1. Multi-component dental cement system which comprises as a first component an ethylenically unsaturated polymerizable compound, as a second component calcium hydroxide or calcium oxide and also a polymerization initiator, the constituents of which may be present in one or more cement components, characterized in that the ethylenically unsaturated polymerizable compound has both at least one β-dicarbonyl group and at least one acrylic, alkacrylic or vinylaromatic group, the cement being cured by chelation of the calcium ions by the β-dicarbonyl compound and free-radical polymerization of the ethylenically unsaturated monomers.

2. Multi-component dental cement system according to Claim 1, characterized in that the alkacrylic group is a methacrylic group and the vinylaromatic group is a styryl group.

3. Multi-component dental cement system according to Claim 1 or 2, characterized in that the ratio of the β-dicarbonyl groups to the ethylenically unsaturated polymerizable groups is in the range from 5:1 to 1:5, in particular 3:1 to 1:3, 1:1 being preferred.

4. Multi-component dental cement system according to one of Claims 1 to 3, characterized in that the ethylenically unsaturated polymerizable compound is a compound of the formula 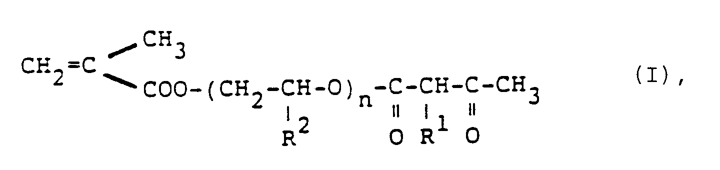 in which
n = 1 to 20, in particular 1 to 10,
R¹ = hydrogen or a C₁- to C₁₀-alkyl group, in particular hydrogen or a C₁- to C₅-alkyl group, such as a methyl group, and
R² = hydrogen or a C₁- to C₁₀-alkyl group, in particular hydrogen or a C₁- to C₅-alkyl group, such as a methyl or ethyl group,
or of the formula 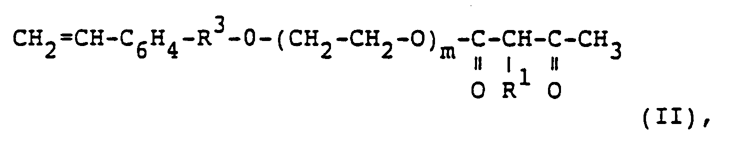 in which
m = 0 to 20, in particular 0 to 10,
R¹ = hydrogen or a C₁- to C₁₀-alkyl group, in particular hydrogen or a C₁- to C₅-alkyl group, such as a methyl group, and
R³ = a C₁- to C₁₀-alkylene group, in particular a C₁- to C₅-alkylene group, such as a methylene group.

5. Multi-component dental cement system according to Claim 4, characterized in that the ethylenically unsaturated polymerizable compound is 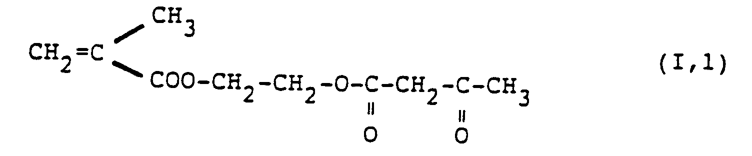 (acetoacetoxyethyl methacrylate, AAEMA) 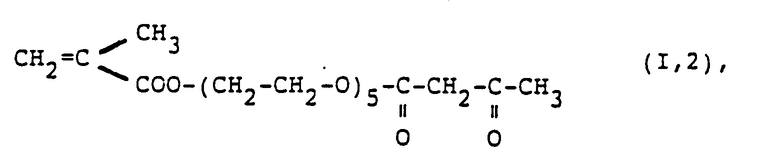 (acetoacetoyl PEG 200 methacrylate, PEG-200-AAMA)
or 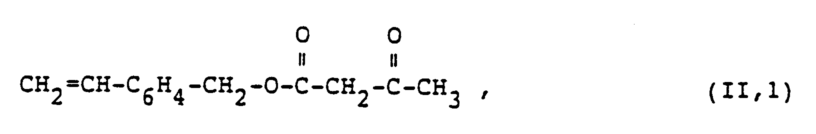 (4-vinylbenzyl acetoacetate, AASt).

6. Multi-component dental cement system according to one of Claims 1 to 5, characterized in that it additionally comprises customary additives, in particular fillers.

7. Multi-component dental cement system according to one of Claims 1 to 6, characterized in that it contains the ethylenically unsaturated polymerizable compound in a quantity from 7 to 70, in particular 7 to 60 and preferably 7 to 55 % by weight.

8. Multi-component dental cement system according to one of Claims 1 to 7, characterized in that the ethylenically unsaturated polymerizable compound contains a number of β-dicarbonyl groups and/or a number of acrylic, alkacrylic or vinylaromatic groups.

9. Process for the production of a multi-component dental cement system according to one of Claims 1 to 8, characterized in that a) a base paste which contains the calcium hydroxide or calcium oxide, and b) an activator paste which contains the ethylenically unsaturated polymerizable compound are initially produced separately and these two pastes are then mixed together.

10. Use of an ethylenically unsaturated polymerizable compound which has both at least one β-dicarbonyl group and at least one acrylic, alkacrylic or vinylaromatic group, for the production of a calcium hydroxide dental cement.

## Revendications

1. Système de ciment dentaire à plusieurs composants, comprenant comme premier composant un composé polymérisable à insaturation éthylénique, comme second composant de l'hydroxyde de calcium ou de l'oxyde de calcium et en outre un initiateur de polymérisation, dont les éléments constitutifs peuvent être contenus dans un ou plusieurs composants du ciment, caractérisé en ce que le composé polymérisable à insaturation éthylénique comporte non seulement au moins un groupe β-dicarbonyle mais encore au moins un groupe acryle, alkacryle ou vinylaromatique, le ciment durcissant par chélation des ions calcium par le composé β-dicarbonyle et polymérisation radicalaire des monomères à insaturation éthylénique.

2. Système de ciment dentaire à plusieurs composants selon la revendication 1, caractérisé en ce que le groupe alkacryle est le groupe méthacryle et le groupe vinylaromatique est le groupe styryle.

3. Système de ciment dentaire à plusieurs composants selon la revendication 1 ou 2, caractérisé en ce que le rapport des groupes β-dicarbonyle aux groupes polymérisables à insaturation éthylénique est dans la plage allant de 5:1 à 1:5, en particulier de 3:1 à 1:3, 1:1 étant préféré.

4. Système de ciment dentaire à plusieurs composants selon une ou plusieurs des revendications 1 à 3, caractérisé en ce que le composant polymérisable à insaturation éthylénique est un composé de formule 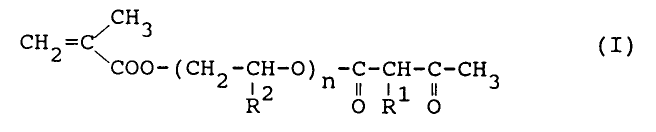 dans laquelle
n = 1 à 20, en particulier 1 à 10,
R¹ est un atome d'hydrogène ou un groupe alkyle en C₁-C₁₀, en particulier un atome d'hydrogène ou un groupe alkyle en C₁-C₅, tel que le groupe méthyle,
R² est un atome d'hydrogène ou un groupe alkyle en C₁-C₁₀, en particulier un atome d'hydrogène ou un groupe alkyle en C₁-C₅, tel que le groupe méthyle ou éthyle,
ou de formule 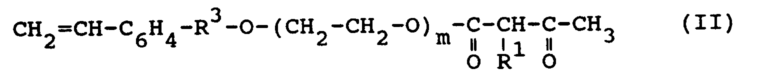 dans laquelle
m = 0 à 20, en particulier 0 à 10,
R¹ est un atome d'hydrogène ou un groupe alkyle en C₁-C₁₀, en particulier un atome d'hydrogène ou un groupe alkyle en C₁-C₅, tel que le groupe méthyle, et
R³ est un groupe alkylène en C₁-C₁₀, en particulier un groupe alkylène en C₁-C₅, tel que le groupe méthylène.

5. Système de ciment dentaire à plusieurs composants selon la revendication 4, caractérisé en ce que le composé polymérisable à insaturation éthylénique est 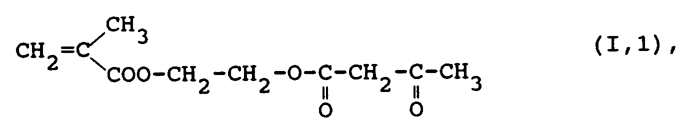 (méthacrylate d'acétoacétoxyéthyle, AAEMA) 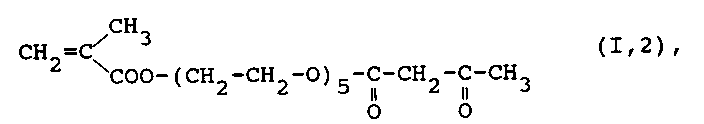 (méthacrylate d'acétoacét-PEG-200, PEG-200-AAMA)
ou 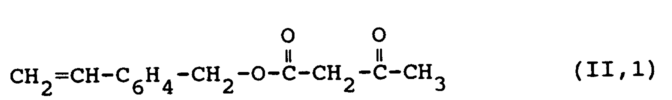 (acétoacétate de 4-vinylbenzyle, AASt)

6. Système de ciment dentaire à plusieurs composants selon l'une des revendications 1 à 5, caractérisé en ce qu'il comprend en outre des additifs usuels, en particulier une charge.

7. Système de ciment dentaire à plusieurs composants selon l'une des revendications 1 à 6, caractérisé en ce qu'il contient le composé polymérisable à insaturation éthylénique en une proportion de 7 à 70, en particulier de 7 à 60 et de préférence de 7 à 55 % en poids.

8. Système de ciment dentaire à plusieurs composants selon l'une des revendications 1 à 7, caractérisé en ce que le composé polymérisable à insaturation éthylénique comporte plusieurs groupes β-dicarbonyle et/ou plusieurs groupes acryle, alkacryle ou vinylaromatiques.

9. Procédé pour la préparation d'un ciment dentaire à plusieurs composants selon l'une des revendications 1 à 8, caractérisé en ce que d'abord on prépare séparément a) une pâte de base qui contient l'hydroxyde de calcium ou l'oxyde de calcium et b) une pâte d'activateur qui contient le composé polymérisable à insaturation éthylénique, et ensuite on mélange ces deux pâtes l'une avec l'autre.

10. Utilisation d'un composé polymérisable à insaturation éthylénique qui comprend non seulement au moins un groupe β-dicarbonyle mais encore au moins un groupe acryle, alkacryle ou vinylaromatique, pour la préparation d'un ciment dentaire à base d'hydroxyde de calcium.
